# EUROPEAN PATENT SPECIFICATION

(11) **EP 1 569 595 B1**
(45) Date of publication and mention of the grant of the patent: **21.05.2008**
(21) Application number: 03776162.4
(22) Date of filing: 10.12.2003
(51) Int. Cl.: A61J 1/00, A61M 1/28, B65D 81/32, A61K 9/08, A61K 31/70

(54) **A METHOD FOR PREPARING A MEDICAL SOLUTION FOR THE MANUFACTURE OF A MEDICAMENT FOR PERITONEAL DIALYSIS**
VERFAHREN ZUR ZUBEREITUNG EINER MEDIZINISCHEN LÖSUNG ZUR HERSTELLUNG EINES MEDIKAMENTS FÜR DIEPERITONEALDIALYSE
PROCEDE DE PREPARATION D'UNE SOLUTION MEDICALE DESTINE A L'ELABORATION D'UN MEDICAMENT POUR DIALYSE PERITONEALE

(30) Priority: 10.12.2002 SE 0203672; 10.12.2002 US 432581 P
(43) Date of publication of application: 07.09.2005
(73) Proprietor: Gambro Lundia AB, 220 10 Lund (SE)
(72) Inventor: CARLSSON, Ola, S-226 49 Lund (SE); LINDEN, Torbjörn, S-290 11 Linderöd (SE); WIESLANDER, Anders, S-222 38 Lund (SE)
(74) Representative: Ahlberg, Lena Camilla
(86) International application number: PCT/SE2003/001921
(87) International publication number: WO 2004/052269

(56) References cited:
- WO-A1-97/06810
- WO-A1-99/27885
- US-A- 5 536 469
- ANDREZJ BREBOROWICZ ET AL: 'Replacement of glucose with N-acetylglucosamine in peritoneal dialysis fluid-experimental study in rats' PERITONEAL DIALYSIS INTERNATIONAL vol. 21, 2001, pages S365 - S367, XP002990428

## Description

### Background of the Invention

The present invention relates to a method for preparing a medical solution, a solution used for preparing the medical solution, a container containing said solution, and use of said solution for the manufacture of a medicament for peritoneal dialysis.

### Background Art

N-acetylglucosamine (NAG) and glucosamine are biochemically classified as amino sugars. Amino sugars are formed in almost all cells from blood glucose through a series of biochemical reactions. Hyaluronan is a polymer composed of dimers containing N-acetylglucosamine and glucuronic acid. It has been shown that the function of the peritoneum as a dialysis membrane is better preserved in rats that have been chronically dialyzed with fluid supplemented with exogenous hyaluronan (see Wieczorowska K, Breborowicz A et al, Protective effect of hyaluronic acid against peritoneal injury, Perit Dial Int 1995; 15:81-83).

Breborowicz A, Kuzlan-Pawlaczyk M et al, The Effect of N-Acetyl-glucosamine as a Substrate for In Vitro Synthesis of Glycosaminoglycans by Human Peritoneal Mesothelial Cells and Fibroblasts, Advances in Peritoneal Dialysis, 1998; 14:31-35, teaches that NAG rapidly stimulates the production of hyaluronan and sulphated glycosaminoglycans by human peritoneal mesothelial cells and fibroblasts.

Wu G, Wieczorowska-Tobis K, et al, N-acetylglucosamine changes permeability of peritoneum during chronic peritoneal dialysis in rats, Perit Dial Int, 1998; 18:217-224 concludes that peritoneal dialysis with a dialysis solution supplemented with N-acetylglucosamin causes accumulation of glucosaminoglycans in the peritoneal interstitium, resulting in a favorable change of the peritoneal permeability.

Breborowicz, M et al (Peritoneal Dialysis International, vol 21, 2001, pages S365-S367, XP002990428) discloses replacement of glucose with N-acetylglucosamine in a peritoneal dialysis fluid which is sterilized by filtration before administration to animals.

U.S. patent No. 5,536,469 discloses a system employing a sterile medical solution containing glucose or glucose-like compounds and a solution intended for said system.

Due to its advantageous characteristics, NAG has been introduced as a component in peritoneal dialysis solutions replacing part or all of the glucose component with a view to obtaining a more biocompatible peritoneal dialysis solution (see WO97/06810).

Peritoneal dialysis is a method for exchanging solutes and water in capillary vessels of a patient's peritoneum with hypertonic solution, which is infused into the peritoneal cavity. The principle of this method is diffusion of solutes transferred according to the concentration gradient and water migration due to osmotic differences. This method has many advantages, e.g. that no special apparatus is commonly required. It gives less influence on the hemodynamics because extracorporeal circulation of the patient's blood is not necessary, and further the peritoneal dialysis is a continuous treatment and therefore more similar to the function of the kidneys.

Peritoneal dialysis is usually classified as continuous ambulatory peritoneal dialysis (CAPD), intermittent peritoneal dialysis (IPD), continuous cyclic peritoneal dialysis (CCPD) or automated peritoneal dialysis (APD).

In CAPD a catheter is permanently implanted in the abdominal wall of the patient and about 1.5 to 2.5 1 of the dialysis fluid is normally introduced via the catheter into the peritoneal cavity. The peritoneal cavity is flooded with this fluid, left for an appropriate lapse of time and then drained. Removal of solutes and water takes place across the peritoneum, which acts as a semipermeable membrane.

The dialysis fluid normally used for peritoneal dialysis is an aqueous solution comprising an osmotic agent such as glucose, electrolytes such as sodium, potassium, calcium, magnesium, and organic acid salts such as sodium lactate, sodium bicarbonate, or sodium pyruvate. The components of these peritoneal dialysis fluids are selected to control the levels of electrolytes or the acid-base equilibrium, to remove waste materials and to efficiently carry out ultrafiltration.

It is known to pack medical solutions in multicompartment bags from e.g. WO 99/27885 (Gambro Lundia AB), in which different solutes may be kept in separate compartments of the bag with a view to, inter alia, regulating the concentration of active ingredients in the finally prepared solution.

Medical fluids are normally sterilised by heat. Medical authorities in many countries require sterilisation after final packaging of the medical product. It is therefore often not possible to sterile filter the solution.

However, a problem with the formation of undesired cytotoxic products during heat sterilisation and storage exists for a variety of medical solutions, inter alia within the dialysis area, e.g. for peritoneal dialysis solutions. It is known e.g. from EP-B1-0 668 785 (Gambro Lundia AB) to reduce the amount of toxic degradation products from glucose or glucose-like compounds in a medical solution.

It has now been found that also amino sugars, e.g. NAG, in conventional medical solutions exhibit an increased cytotoxicity after heat sterilisation. This cytotoxicity depends on the formation of toxic degradation products from said amino sugars. In contrast to glucose, none of the known glucose degradation products has been found in heat sterilised NAG solutions. This fact has not been known previously and forms the basis for the present invention.

NAG and other amino sugars have a major difference from glucose and glucose-like compounds by having one amino group and possibly an acetyl group coupled to the glucose ring. Regarding the degradation process, it has been found that the pH of a NAG solution increases during sterilisation while in the case of glucose it decreases during sterilisation. This indicates that NAG is, in contrast to glucose, degraded by a hydrolysis that forms acetate, which increases the pH.

Thus, there is a need to solve the above defined problem and to provide a medical solution containing amino sugars, in particular NAG, and derivatives thereof and at the same time having the ability to be heat sterilised without the formation of the above-mentioned cytotoxic products.

### Summary of the Invention

The object of the present invention is to solve the above-mentioned problem.

According to the present invention this object is achieved by an improved method for preparing a medical solution, preferably a peritoneal dialysis solution, comprising the steps of:
a) providing a solution comprising N-acetylglucosamine (NAG) in at least one compartment of a container in a concentration of 20-40% by weight, with the basis of the solution in said at least one compartment, and
b) terminal sterilisation of said at least one compartment and the contents therein.

Further, the present invention relates to the solution used for preparing the medical solution, and to a container containing said solution.

The present invention also relates to use of said solution for the manufacture of a medicament for peritoneal dialysis.

In another aspect the present invention relates to peritoneal dialysis, wherein said dialysis comprises the introduction of said medicament for peritoneal dialysis into the peritoneal cavity of a patient.

Further disclosure of the objects, problems, solutions and features of the present invention will be apparent from the following detailed description of the invention with reference to the drawings and the appended claims.

### Brief Description of the Drawings

Fig. 1 is a graph showing the relationship between the inhibition of cell growth (ICG) and the NAG concentration in a solution containing NAG during sterilisation.
Fig. 2 is a bar diagram showing the effect of increased NAG concentration of 1.5% and 30% for three different pH values.
Fig. 3 is a graph showing the fluorescence of heat sterilised NAG containing solutions at different pH values.
Fig. 4 is a graph showing the relationship between pH and inhibition of cell growth (ICG) in a solution containing 1.5% NAG.
Figs. 5a - 5d are HPLC chromatograms showing the different decomposition patterns between a heat sterilised NAG solution and a heat sterilised glucose solution.

### Detailed Description of Preferred Embodiments

The present invention is a development of the above mentioned teachings and relates to a method for preparing a sterile medical solution, preferably a solution for peritoneal dialysis.

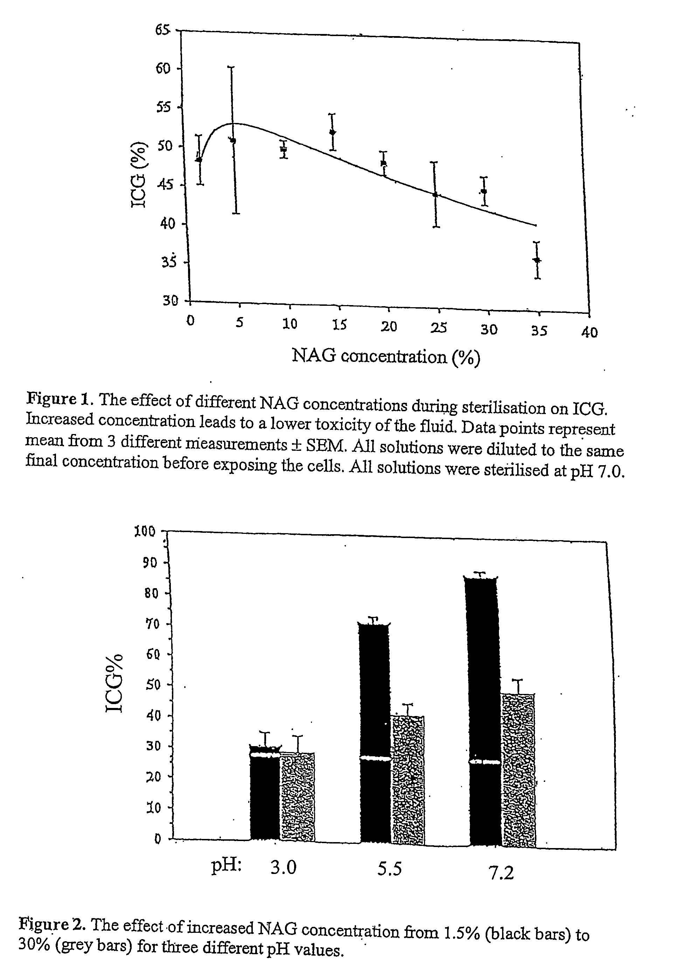

Experiments in which measurements of the percentage of inhibition of cell growth (ICG) and fluorescence have been made at varying NAG concentrations, and also at varying pH values, during terminal heat sterilisation are illustrated in Figs 1-4. The results of the experiments imply that the concentration of the NAG should be optimised. The results also show that, in a preferred embodiment of the invention, also the pH of the NAG containing solution should be decreased from the neutral level.

A simple, reliable and known way to study the cytotoxicity of substances or of medical fluids is to test proliferation as in vitro inhibition of cell growth (ICG) in cultured cells. Another method to get a rough estimation of the amount of NAG that is rearranged is measurement of the fluorescence.

More precisely, from the graph in Fig. 1 it can be seen that the inhibition of cell growth reaches a maximum with sterilisation of a solution containing about 5% NAG. with increasing NAG concentration the inhibition of cell growth decreases.

The bar diagram in Fig. 2 illustrates the effect of increased NAG concentration at different pH values during terminal heat sterilisation. It can be seen that the percentage inhibition of cell growth is lower after terminal heat sterilisation at a pH of 3.0 than at a pH of 5.5 and 7.2, and that the inhibition of cell growth is lower after a terminal heat sterilisation at a NAG concentration of 30% than at a NAG concentration of 1.5% for all three pH values.

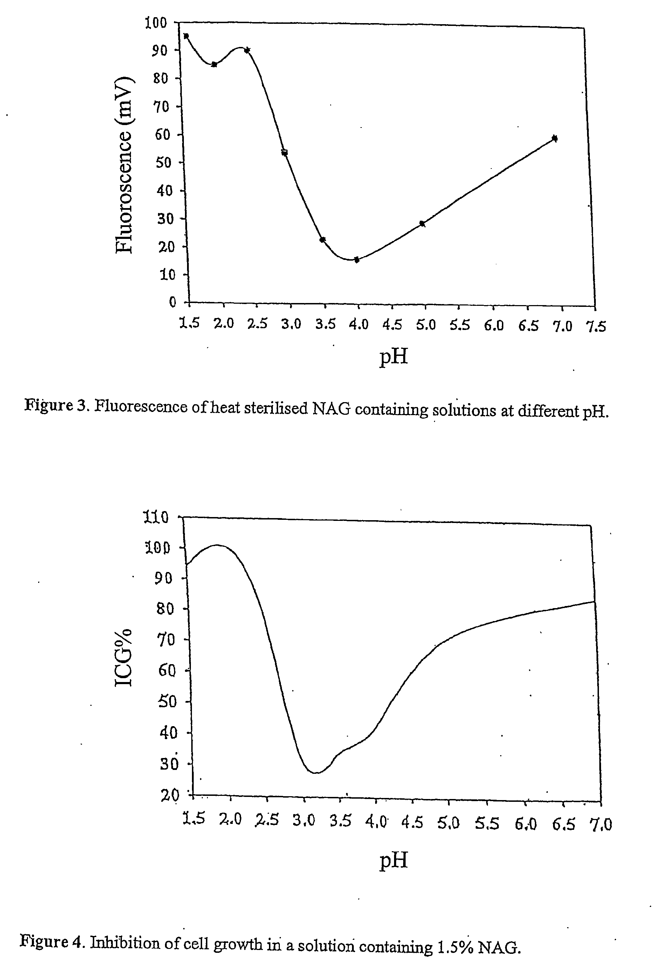

Fig. 3 shows the relationship between pH for a terminally heat sterilised NAG containing solution and the fluorescence, measured at an excitation at 350 nm and an emission of 430 nm. The lowest fluorescence is seen at around pH 4.

Fig. 4 shows the inhibition of cell growth in a solution containing 1.5% NAG.

It has also been found that a NAG containing solution sterilised at an optimal pH around 2.5-3.5 from an in vitro toxicological point of view is more compatible for humans than solutions giving a higher percentage of inhibited growth when sterilised at higher or lower pH values.

The term "terminal" sterilisation used herein is intended to mean that the product is sterilised in its final package by a sterilisation method involving addition of energy, e.g. heating and/or radiation (see also European Pharmacopoeia 1977, p 283, col 1, last paragraph, to col 2, first paragraph, and p 284, col 2, "Filtration", for a detailed description of this term, as well as for a review of different sterilisation techniques). Sterile filtration involves filtration of the solution to be included in the product, and the solution is aseptically filled into the container. This does not secure the sterility required in this context, and sterile filtration can therefore not be used as a sterilisation method for a medicament if it is possible to sterilise the medicament by terminal sterilisation.

The terminal sterilisation may include heat sterilisation and/or radiation sterilisation, but is preferably heat sterilisation effected in an autoclave at a temperature of at least 100°C, preferably at 121°C. The sterilisation time may vary depending on the sterilisation temperature, the type of container and the contents therein to be sterilised. The radiation sterilisation may be either ionizing or non-ionizing sterilisation. Examples of ionising sterilisation are gamma and beta radiation. An example of non-ionizing radiation sterilisation is UV radiation.

The method according to the present invention is preferably effected for a multicompartment container as disclosed in WO 99/27885 (Gambro AB). In the present invention, such a container comprises at least one compartment containing a physiologically compatible pH adjusting and diluting solution as well as at least one compartment containing a solution comprising N-acetylglucosamine, in the following called NAG for simplicity. The NAG solution may be present in only one compartment. The solutions in the different compartments are heat sterilisable, and the whole container can be heat sterilised in an autoclave with the solutions in situ in said compartments. The solutions in the separated compartments, which are delimited from each other during the sterilisation and the subsequent storage, can be mixed after sterilisation to form a finally prepared sterile medical solution, preferably a solution for peritoneal dialysis. It may also be mixed with a terminally sterilised pH adjusting and diluting solution in at least one other terminally sterilised compartment of the container, thereby finally preparing the medical solution. Such a medical solution may be stored after terminal sterilisation up to longer periods of time before mixing with the terminally sterilised pH adjusting and diluting solution. The terminal sterilisation can however also be effected for separated interconnectable containers comprising the solutions to sterilise and provided with connection means with sterile connecting valves for sterile connection. The separated containers can also be connected already during manufacture by means of a breakable seal, for example a conventional frangible pin.

In one preferred embodiment of the invention, the NAG in the NAG containing solution present in one or more of said compartments, is present in a concentration of 15-40% by weight with the basis on the solution in each of said compartments, e.g. 15, 20, 25, 30, 35, and 40% by weight.

In another embodiment of the invention the medical solution comprising NAG in a concentration of 15-40% by weight, preferably 20-40% by weight, most preferably at least 30% by weight, has a pH of 2.0-5.0, preferably 2.5-3.5, most preferably 3.0, wherein the formation of cytotoxic substances during the terminal sterilisation step is substantially prevented.

The upper limit for the concentration of NAG in the solution is determined by its solubility therein.

The compartment comprising the NAG containing solution may also contain any organic acid or other pH stabilising agent in order to further stabilise the pH during sterilisation. The solutions of the different compartments have such respective pH values, concentrations and volumes that the finally prepared medical solution after mixing the solutions of the compartments has a pH that is substantially neutral, i.e. a pH between 6.0 and 8.0, preferably about 7.4, and a NAG concentration between 0.2 and 15.0%, preferably 0.5-6.0%, e.g. 0.5-2.0% by weight, with the basis on the finally prepared solution.

The volume of each compartment, as well as the proportion between the compartments, is in practice not critical. Each compartment volume depends on the volume of constituent to be present therein. In the most preferred embodiment, the compartment which accommodates the pH adjusting and diluting solution is larger than the compartment which accommodates the NAG containing solution and is also the compartment in which the solution/solutions from the other compartments is/are mixed with the pH adjusting and diluting solution.

In a preferred embodiment the medicament to be prepared is a peritoneal dialysis solution containing N-acetylglucosamine (NAG) and having a pH of 7.4.

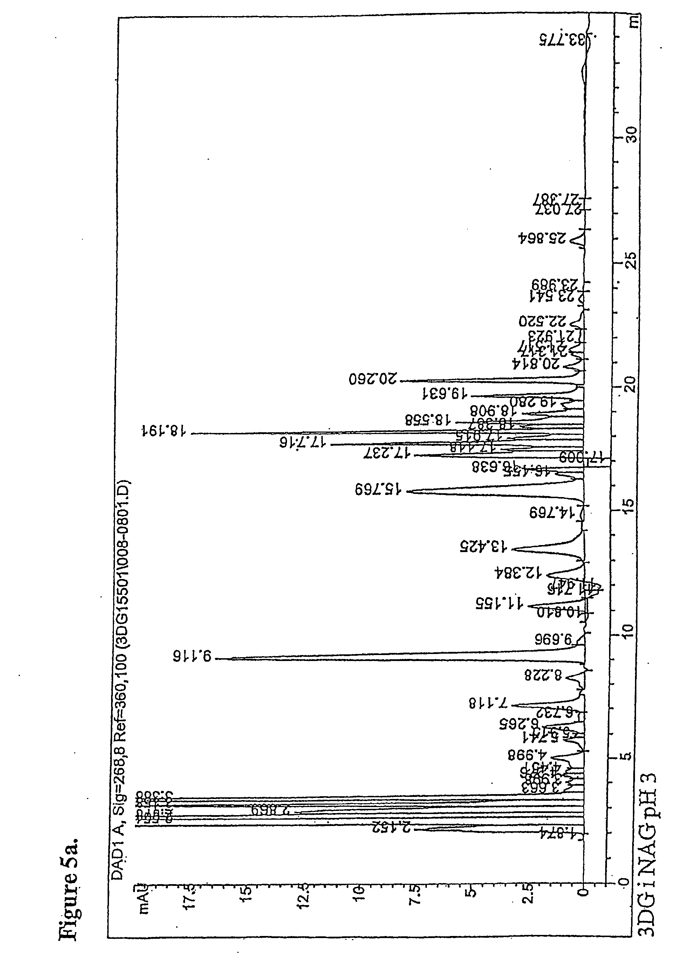

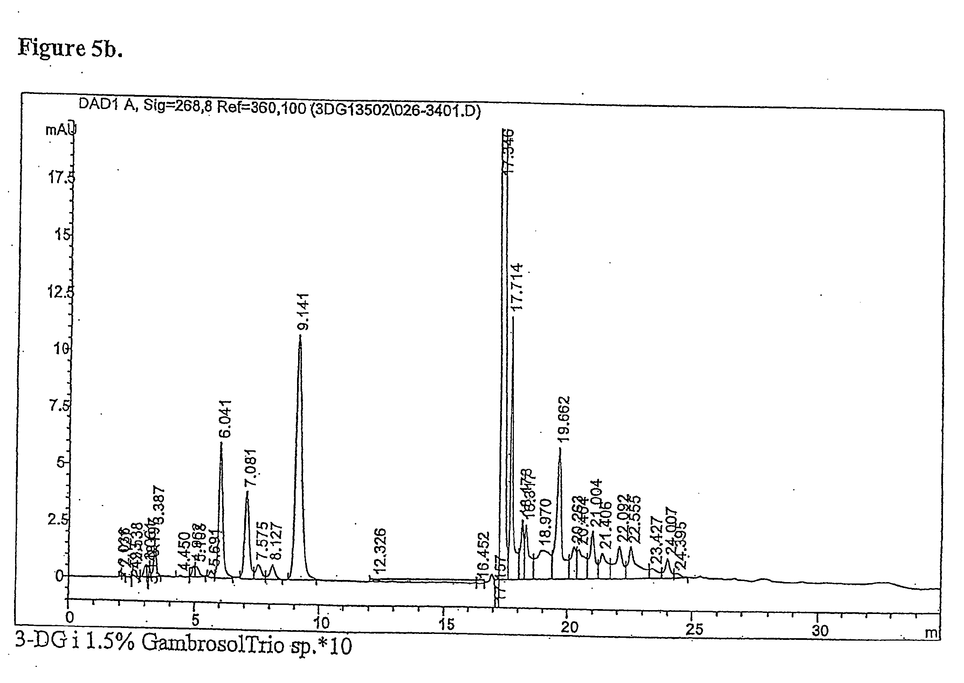

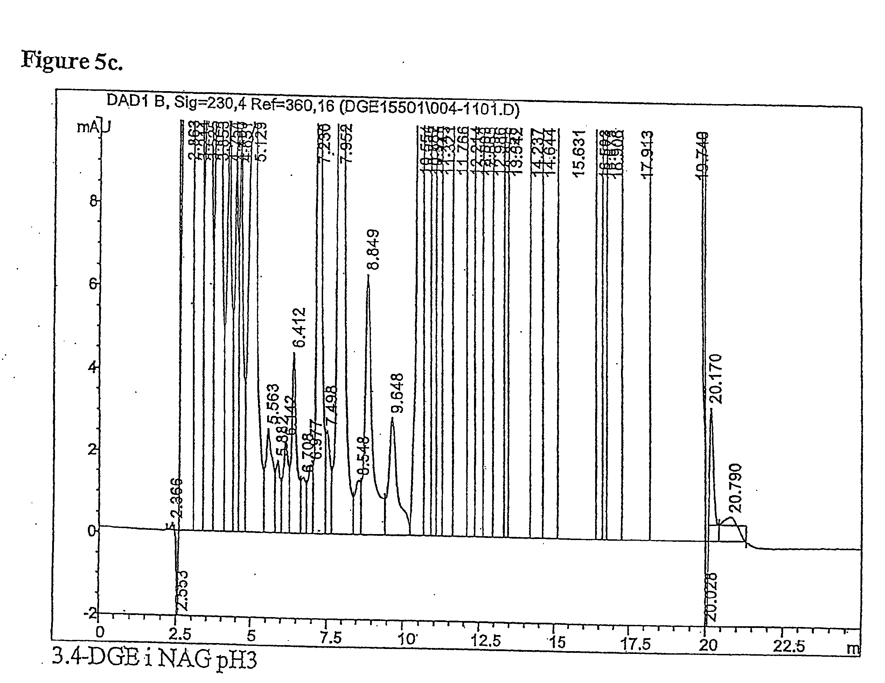

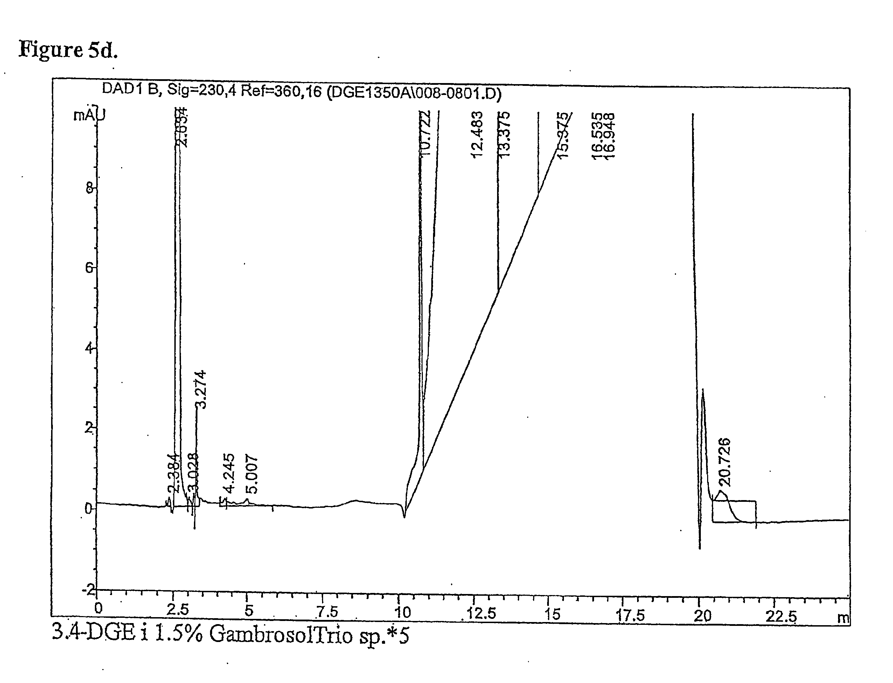

It should be noted that the decomposition pattern for the NAG solution during heat sterilisation follows specific Maillard reactions giving several different toxic decomposition products. The decomposition pattern differs for e.g. a glucose solution during heat sterilisation, wherein different decomposition products are formed. This difference is illustrated in Figs 5a - 5d, showing chromatograms when analysing 3-DG and 3,4-DGE in a NAG solution and in a glucose solution, respectively. The difference in degradation is likely due to that the NAG molecules contains groups that allows the Maillard reaction to take place and therefore create a large number of degradation products not seen in a glucose solution.

Other known amino sugars in this context and comprising one or more acetylated or deacetylated amino sugars are galactosamine, N-acetylgalactosamine, mannosamine, and N-acetyl-mannosamine in the form of monomers, oligomers and/or polymers thereof including chitin, and human glucosaminoglycans, as well as derivatives thereof. The amino sugar according to the present invention is N-acetylglucosamine (NAG).

The term "derivatives thereof" used herein means derivatives of said amino sugars having the same or essentially the same ability to form cytotoxic degradation products during sterilisation.

The term "pH adjusting and diluting solution" used herein means a solution to be mixed with, e.g. acting as a receiving medium for, the NAG containing solution and at the same time a solution adjusting the pH of the solution after mixing with the NAG containing solution to essentially neutral, i.e. with a pH between for example 6.0 and 8.0, preferably about 7.4.

The term "low levels of cytotoxic degradation products" used herein means that the amount of degradation products from the NAG is so low in the medical solution prepared according to the present invention that it is not more toxic to cultured cells than dialysis solutions according to prior art.

The pH adjusting and diluting solution in the preferred embodiment contains pH adjusting agents, such as salts of inorganic acids, organic acids, alkalic substances in a pharmaceutically stable range. Inorganic acids include hydrochloride acid., organic acids include lactic acid, malic acid, acetic acid, succinic acid, maleic acid, pyruvic acid, citric acid, gluconic acid., and alkalic substances include sodium hydrate, sodium bicarbonate. Also, various amino acids can be used as a pH-adjusting agent.

After sterilisation the NAG containing solution is finally prepared for use by mixing it with the pH adjusting and diluting solution, optionally with solutions in other compartments of the container. The medical solution, preferably a peritoneal dialysis solution, thus obtained may also comprise different electrolyte ions, e.g. sodium, potassium, calcium, magnesium, chloride, lactate, and bicarbonate ions, in concentrations which are biocompatible and substantially isotonic. The electrolytes may be originally present in the pH adjusting and diluting solution, the NAG containing solution and/or another solution in one or more other compartments of the container, depending on their compatibility during sterilisation and storage, normally in the form of pharmaceutically acceptable salts. The amount of cations in a peritoneal dialysis solution ready for use is generally 110 to 140 mEq/ml of sodium ions, 0 to 0.05 mEq/l of potassium ions, 0 to 3 mEq/l of magnesium ions and 0 to 6 mEq/l of calcium ions. Preferably the amount of chloride ions is 80 to 144 mEq/l.

The peritoneal dialysis solution as a preferred embodiment of the medical solution according to the present invention may also comprise other physiologically compatible constituents, e.g. further osmotic agents, such as carbohydrates, preferably glucose, proteins and peptides, preferably albumin, as well as antioxidants, such as bisulphite.

The peritoneal dialysis solution of the present invention described above is applicable not only to continuous ambulatory peritoneal dialysis (CAPD) but also to intermittent peritoneal dialysis (IPD), continuous cyclic peritoneal dialysis (CCPD), and automated peritoneal dialysis (APD). Moreover, it contains low levels of cytotoxic degradation products from amino sugars.

The present invention also relates to a solution as such having the above defined characteristics.

The present invention also relates to a container containing the NAG solution in at least one compartment, wherein said solution optionally has been sterilised and contains low levels of cytotoxic degradation products.

Further, the present invention relates to use of the solution according to the present invention for the manufacture of a medicament for peritoneal dialysis, wherein it is mixed with a sterilised pH adjusting and diluting solution.

As stated above, the present invention also relates to a method of performing peritoneal dialysis, wherein said method comprises the introduction of said medicament for peritoneal dialysis into the peritoneal cavity of a patient.

In order to illustrate different embodiments of the present invention, containers having different compartment constructions containing the constituents for the preparation of a peritoneal dialysis solution will be described in the Examples below, as well as the composition of the solutions in each compartment. In the examples, N-acetylglucoseamine (NAG) is used as amino sugar, either in one or two compartments of the container. The pH for the NAG containing solutions in each compartment varied between 2.0 and 5.0 before mixing and between 6.0 and 8.0 in the finally prepared medical solution.

### Examples

### Example 1

| | | |
|---|---|---|
| Compartment A | Volume | 100 ml |
| | Sodium | 0-140 mM |
| | NAG | 300 g/l |
| Compartment B | Volume | 180 ml |
| | Sodium | 0-140 mM |
| | NAG | 300 g/l |
| Compartment C | Volume | 1900 ml |
| | Sodium | 0-140 mM |
| | Lactate | 40 mM |
| | Magnesium | 0.25-0.75 mM |
| | Calcium | 0.9-2.0 mM |

Final composition when the contents of compartments A+C are mixed:

| | |
|---|---|
| Volume | 2000 ml |
| Sodium | 0-140 mM |
| NAG | 15 g/l |
| Lactate | 38 mM |
| Magnesium | 0.24-0.71 mM |
| Calcium | 0.85-1.9 mM |

Final composition when the contents of compartments B+C are mixed:

| | |
|---|---|
| Volume | 2080 ml |
| Sodium | 0-140 mM |
| NAG | 26 g/l |
| Lactate | 36.5 mM |
| Magnesium | 0.22-0.68 mM |
| Calcium | 0.82-1.8 mM |

Final composition when the contents of compartments A+B+C are mixed:

| | |
|---|---|
| Volume | 2180 ml |
| Sodium | 0-140 mM |
| NAG | 38.5 g/l |
| Lactate | 34.9 mM |
| Magnesium | 0.21-0.65 mM |
| Calcium | 0.78-1.7 mM |

### Example 2

| | | |
|---|---|---|
| Compartment A | Volume | 100 ml |
| | Sodium | 0-140 mM |
| | NAG | 300 g/l |
| Compartment B | Volume | 180 ml |
| | Sodium | 0-140 mM |
| | Glucose | 500 g/l |
| Compartment C | Volume | 1900 ml |
| | Sodium | 0-140 mM |
| | Lactate | 40 mM |
| | Magnesium | 0.25-0.75 mM |
| | Calcium | 0.9-2.0 mM |

Final composition when the contents of compartments A+C are mixed:

| | |
|---|---|
| Volume | 2000 ml |
| Sodium | 0-140 mM |
| NAG | 15 g/l |
| Glucose | 0 g/l |
| Lactate | 38 mM |
| Magnesium | 0.24-0.71 mM |
| Calcium | 0.86-1.9 mM |

Final composition when the contents of compartments A+B+C are mixed:

| | |
|---|---|
| Volume | 2100 ml |
| Sodium | 0-140 mM |
| NAG | 14.3 g/l |
| Glucose | 23.8 g/l |
| Lactate | 36 mM |
| Magnesium | 0.23-0.68 mM |
| Calcium | 0.81-1.8 mM |

### Example 3

| | | |
|---|---|---|
| Compartment A | Volume | 60 ml |
| | Sodium | 0-140 mM |
| | NAG | 200 g/l |
| | Glucose | 330 g/l |
| Compartment B | Volume | 100 ml |
| | Sodium | 0-140 mM |
| | NAG | 200 g/l |
| | Glucose | 330 g/l |
| Compartment C | Volume | 1900 ml |
| | Sodium | 0-140 mM |
| | Lactate | 40 mM |
| | Magnesium | 0.25-0.75 mM |
| | Calcium | 0.9-2.0 mM |

Final composition when the contents of compartments A+C are mixed:

| | |
|---|---|
| Volume | 1960 ml |
| Sodium | 0-140 mM |
| NAG | 6.2 g/l |
| Glucose | 10.1 g/l |
| Lactate | 38.8 mM |
| Magnesium | 0.24-0.73 mM |
| Calcium | 0.87-1.9 mM |

Final composition when the contents of compartments B+C are mixed:

| | |
|---|---|
| Volume | 2000 ml |
| Sodium | 0-140 mM |
| NAG | 10.0 g/l |
| Glucose | 16.5 g/l |
| Lactate | 38.0 mM |
| Magnesium | 0.24-0.71 mM |
| Calcium | 0.86-1.9 mM |

Final composition when the contents of compartments A+B+C are mixed:

| | |
|---|---|
| Volume | 2060 ml |
| Sodium | 0-140 mM |
| NAG | 15.5 g/l |
| Glucose | 25.6 g/l |
| Lactate | 37 mM |
| Magnesium | 0.23-0.69 mM |
| Calcium | 0.83-1.8 mM |

The invention has been described above with reference to preferred embodiments of the invention. A skilled person will recognise that further combinations are possible. Modifications which are apparent to a skilled person are intended to be incorporated within the scope of the invention, which is limited only by the appended claims.

## Claims

1. A method for preparing a medical solution, comprising the steps of:
a) providing a solution comprising N-acetylglucosamine (NAG) in at least one compartment of a container in a concentration of 15-40% by weight, with the basis of the solution in said at least one compartment, and
b) terminal sterilisation of said at least one compartment and the contents therein.

2. The method according to claim 1, wherein said N-acetylglucosamine NAG in said at least one compartment of said container is provided in a concentration of 20-40% by weight, preferably at least 30% by weight, with the basis of the solution in said at least one compartment.

3. The method according to any one of the preceding claims, wherein the solution comprising N-acetylglucosamine (NAG) in said at least one compartment of said container is provided at a pH of 2.0-5.0.

4. The method according to claim 3, wherein said pH is 2.5-3.5, preferably 3.0.

5. The method according to any one of the preceding claims, wherein the terminal sterilisation is heat sterilisation at a temperature of at least 100°C, preferably 121°C, and/or radiation sterilisation.

6. The method according to any one of the preceding claims, wherein each compartment of the container is delimited from the other/others during the terminal sterilisation, and wherein the terminally sterilised solution containing N-acetylglucosamine (NAG) is mixed with a terminally sterilised pH adjusting and diluting solution in at least one other terminally sterilised compartment of the container, thereby finally preparing the medical solution.

7. The method according to claim 6, wherein the pH in the finally prepared medical solution is 6.0-8.0, preferably 7.4.

8. The method according to claim 6 or 7, wherein the concentration of N-acetylglucosamine (NAG) in the finally prepared solution is 0.2-15.0% by weight, preferably 0.5-6.0% by weight.

9. The method according to any one of the preceding claims, wherein physiologically compatible constituents in the form of carbohydrates, preferably glucose, proteins, peptides, and antioxidants are present in one or more of said compartments.

10. The method according to any one of the preceding claims, wherein the medical solution prepared is a peritoneal dialysis solution.

11. A solution comprising N-acetylglucosamine (NAG) in an amount of 15-40% by weight, preferably 20-40% by weight, most preferably at least 30% by weight, wherein said solution is terminally sterilised and contains low levels of cytotoxic degradation products.

12. The solution according to claim 11, wherein the pH of the solution is 2.0-5.0, preferably 2.5-3.5, most preferably 3.0.

13. A container comprising at least one compartment containing a solution according to claim 11 or 12.

14. Use of a solution according to claim 11 or 12 for the manufacture of a medicament for peritoneal dialysis, wherein it is mixed with a terminally sterilised pH adjusting and diluting solution.

## Patentansprüche

1. Verfahren zum Herstellen einer medizinischen Lösung, mit den Schritten, dass:
a) eine Lösung, die N-Acetylglukosamin (NAG) umfasst, in zumindest einer Kammer eines Behälters in einer Konzentration von 15 bis 40 Gew.-% vorgesehen wird, wobei sich die Basis der Lösung in der zumindest einen Kammer befindet, und
b) die zumindest eine Kammer und die Inhalte darin endsterilisiert werden.

2. Verfahren nach Anspruch 1,
wobei das N-Acetylglukosamin NAG in der zumindest einen Kammer des Behälters in einer Konzentration von 20 bis 40 Gew.-%, bevorzugt zumindest 30 Gew.-% vorgesehen wird, wobei sich die Basis der Lösung in der zumindest einen Kammer befindet.

3. Verfahren nach einem der vorhergehenden Ansprüche,
wobei die Lösung, die N-Acetylglukosamin (NAG) umfasst, in der zumindest einen Kammer des Behälters mit einem pH von 2,0 bis 5,0 vorgesehen ist.

4. Verfahren nach Anspruch 3,
wobei der pH 2,5 bis 3,5, bevorzugt 3,0 beträgt.

5. Verfahren nach einem der vorhergehenden Ansprüche,
wobei die Endsterilisierung eine Wärmesterilisierung bei einer Temperatur von zumindest 100°C, bevorzugt 121°C und/oder eine Strahlungssterilisierung ist.

6. Verfahren nach einem der vorhergehenden Ansprüche,
wobei jede Kammer des Behälters von der bzw. den anderen während der thermischen Sterilisierung abgegrenzt wird, und wobei die thermisch sterilisierte Lösung, die N-Acetylglukosamin (NAG) enthält, mit einer endsterilisierten, pH-einstellenden und verdünnenden Lösung in zumindest einer anderen thermisch sterilisierten Kammer des Behälters gemischt wird, wodurch die medizinische Lösung endgültig hergestellt wird.

7. Verfahren nach Anspruch 6,
wobei der pH in der endgültig hergestellten medizinischen Lösung 6,0 bis 8,0, bevorzugt 7,4 beträgt.

8. Verfahren nach einem der Ansprüche 6 oder 7,
wobei die Konzentration von N-Acetylglukosamin (NAG) in der endgültig hergestellten Lösung 0,2 bis 15,0 Gew.-%, bevorzugt 0,5 bis 6,0 Gew.-% beträgt.

9. Verfahren nach einem der vorhergehenden Ansprüche,
wobei physiologisch kompatible Bestandteile in der Form von Carbohydraten, bevorzugt Glykose, Proteinen, Peptiden und Antioxidantien in einer oder mehreren der Kammern vorhanden sind.

10. Verfahren nach einem der vorhergehenden Ansprüche,
wobei die medizinische Lösung, die hergestellt wird, eine Peritonealdialyselösung ist.

11. Lösung, die N-Acetylglukosamin (NAG) in einer Menge von 15 bis 40 Gew.-%, bevorzugt 20 bis 40 Gew.-%, am bevorzugtesten zumindest 30 Gew.-% umfasst, wobei die Lösung thermisch sterilisiert ist und geringe Niveaus an zytotoxischen Abbauprodukten enthält.

12. Lösung nach Anspruch 11,
wobei der pH der Lösung 2,0 bis 5,0, bevorzugt 2,5 bis 3,5 und am bevorzugtesten 3,0 beträgt.

13. Behälter mit zumindest einer Kammer, die eine Lösung gemäß Anspruch 11 oder 12 enthält.

14. Verwendung einer Lösung gemäß Anspruch 11 oder 12 zur Herstellung eines Medikaments zur Peritonealdialyse, wobei es mit einer thermisch sterilisierten, pH-einstellenden und verdünnenden Lösung gemischt ist.

## Revendications

1. Procédé pour préparer une solution médicale, comportant les étapes consistant à :
a) fournir une solution comprenant une N-acétylglucosamine (NAG) dans au moins un compartiment d'un récipient dans une concentration de 15 à 40 % en poids sur la base de la solution dans ledit compartiment, et
b) stériliser finalement ledit au moins un compartiment et ses contenus.

2. Procédé selon la revendication 1, **caractérisé en ce que** ladite N-acétylglucosamine (NAG) dans ledit au moins un compartiment dudit récipient est fournie dans une concentration de 20 à 40 % en poids, de préférence au moins 30% en poids sur la base de la solution dans ledit au moins un compartiment.

3. Procédé selon l'une quelconque des revendications précédentes, **caractérisé en ce que** la solution comportant la N-acétylglucosamine (NAG) dans ledit au moins un compartiment dudit récipient possède un pH de 2.0 à 5.0.

4. Procédé selon la revendication 3, **caractérisé en ce que** ledit pH est de 2.5 à 3.5, de préférence 3.0.

5. Procédé selon l'une quelconque des revendications précédentes, **caractérisé en ce que** la stérilisation finale est une stérilisation par chauffage à une température d'au moins 100°C, de préférence, 121°C, et/ou une stérilisation par irradiation.

6. Procédé selon l'une quelconque des revendications précédentes, **caractérisé en ce que** chaque compartiment du récipient est délimité de l'autre ou des autres compartiments durant la stérilisation finale, et **en ce que** la solution finalement stérilisée contenant le N-acétylglucosamine (NAG) est mélangée avec une solution de dilution et d'ajustement du pH finalement stérilisée dans au moins un autre compartiment finalement stérilisé du récipient, en préparant ainsi finalement la solution médicale.

7. Procédé selon la revendication 6, **caractérisé en ce que** le pH de la solution médicale finalement préparée est de 6.0 à 8.0, de préférence 7.4.

8. Procédé selon l'une des revendications 6 ou 7, **caractérisé en ce que** la concentration en N-acétylglucosamine (NAG) dans la solution finalement préparée est de 0.2 à 15.0 % en poids, de préférence de 0.5 à 6.0 % en poids.

9. Procédé selon l'une quelconque des revendications précédentes, **caractérisé en ce que** des constituants physiologiquement compatibles sous la forme d'hydrate de carbone, de préférence du glucose, des protéines, des peptides, et des antioxydants sont présents dans un ou plusieurs desdits compartiments.

10. Procédé selon l'une quelconque des revendications précédentes, **caractérisé en ce que** la solution médicale préparée est une solution de dialyse péritonéale.

11. Solution comportant de la N-acétylglucosamine (NAG) dans une quantité de 15 à 40% en poids, de préférence 20 à 40 % en poids, de préférence au moins 30 % en poids, **caractérisée en ce que** ladite solution est finalement stérilisée et contient des faibles niveaux de produits de dégradation cytotoxique.

12. Solution selon la revendication 11, **caractérisée en ce que** le pH de la solution est de 2.0 à 5.0, de préférence 2.5 à 5.3, de préférence encore 3.0.

13. Récipient comportant au moins un compartiment contenant une solution selon la revendication 11 ou 12.

14. Utilisation d'une solution selon la revendication 11 ou 12 pour la production d'un médicament pour la dialyse péritonéale, **caractérisé en ce qu'**elle est mélangée avec une solution de dilution et d'ajustement du pH finalement stérilisée.
